# EUROPEAN PATENT APPLICATION

(11) **EP 3 434 651 A1**
(43) Date of publication of application: **30.01.2019**
(21) Application number: 17769649.9
(22) Date of filing: 27.01.2017
(51) Int. Cl.: C02F 11/04, A61L 2/10, A61L 11/00, B09B 3/00, C02F 11/00, C02F 11/02, C12M 1/00, C12M 1/02, C12M 1/113, C12M 1/12

(54) **METHANE FERMENTATION SYSTEM**

(30) Priority: 24.03.2016 JP 2016060637
(71) Applicant: Price Management Of Japan Co., Ltd., Kitakyushu-shi, Fukuoka 808-0135 (JP)
(72) Inventor: JI, Hezhe, Kitakyushu-shi Fukuoka 808-0135 (JP); ISHIBASHI, Yasuhiro, Kitakyushu-shi Fukuoka 808-0135 (JP); NAKAMICHI, Takahiro, Kitakyushu-shi Fukuoka 808-0135 (JP); KAWAKAMI, Shigeki, Kitakyushu-shi Fukuoka 808-0135 (JP)
(74) Representative: V.O.
(86) International application number: PCT/JP2017/002894
(87) International publication number: WO 2017/163602

(57) **Abstract**

A methane fermentation system (1) includes a successive treatment line (5) that includes: a solubilization tank (12) that is contained in a container (2), in which tank a solubilization treatment is carried out on an organic treated material; and a methane fermentation tank (13) contained in a container (3), in which tank methane is produced by fermenting the treated material that has been solubilized in the solubilization tank (12). An organic waste material is sent to a mohno pump with crusher (10), a mixing tank (11), the solubilization tank (12), and the methane fermentation tank (13) in the order mentioned, while undergoing a solubilization treatment and then methane fermentation to produce and output methane gas. The methane fermentation tank (13) is provided with a heating mechanism that uses heat or the like generated in the solubilization tank (12).

## Description

### Technical Field

The present disclosure relates to a methane fermentation system.

### Background Art

In recent years, electricity production called biomass power generation, in which electric power is generated by using methane gases produced through methane fermentation treatment on organic waste materials such as sewage sludge, animal excrement, and kitchen waste, has been actively carried out (see Patent Literature 1, for example).

For efficient methane fermentation, equipment for solubilization treatment is needed that solubilizes, prior to methane fermentation, an organic waste material to the extent that hardly-degradable solid organic matter included in the organic waste material can be used as a substrate for microorganisms. In addition, for efficient solubilization treatment, other pieces of equipment are needed including the equipment for crushing an organic waste material into pieces as small as possible and the equipment for pre-treatment of the crushed organic waste material to create an environment suitable for microorganisms to solubilize the organic waste material.

### Citation List

### Patent Literature

Patent Literature 1: Unexamined Japanese Patent Application Kokai Publication No. 2015-165019

### Summary of Invention

### Technical Problem

Since various pieces of equipment are needed for methane fermentation as described above, a methane gas generation system is usually large in size. As a result, the installation takes much time with difficulties in constructing the right equipment in the right place. This is one of the reasons biomass power generation is hindered from becoming widespread.

The present disclosure has been created in view of the foregoing circumstances, and an objective of the disclosure is to provide a methane fermentation system that can be easily moved and allows the right equipment to be placed in the right place.

### Solution to Problem

To achieve the above-described objective, a methane fermentation system of the present disclosure includes a successive treatment line, the treatment line including:
a solubilization tank contained in a container, in which tank a solubilization treatment is carried out on an organic treated material; and
a methane fermentation tank contained in a container, in which tank methane is produced by fermenting the treated material that has been solubilized in the solubilization tank,
wherein the solubilization tank includes:
an inner container that contains a treated material; and
an outer container that is disposed outside the inner container and contains a heating medium heated to a predetermined temperature by heat generated by the solubilization treatment on the treated material contained in the inner container, the heating medium being contained between the outer container and the inner container, and
wherein the methane fermentation system includes:
a treated material circulator that circulates a treated material by taking out part of the treated material contained in the methane fermentation tank from the methane fermentation tank and returning the part of the treated material to an inside;
a heating medium circulator that circulates the heating medium by taking out part of the heating medium from the solubilization tank and returning the part of the heating medium to an inside; and
a heat exchanger that exchanges heat between the heating medium circulating in the heating medium circulator and the treated material circulating in the treated material circulator.

The successive treatment line may include:
a crusher that is contained in the container and crushes a plurality of different organic waste materials that have been charged; and
a mixing tank that is contained in the container, and contains and mixes the crushed organic waste materials as the treated material to be sent to the solubilization tank.

The successive treatment line may include:
an ultraviolet light irradiator that irradiates the treated material contained in the mixing tank with ultraviolet light.

The successive treatment line may include:
a solubilizing bacteria supplier that supplies solubilizing bacteria to the treated material contained in the solubilization tank.

The solubilization tank may be contained in a first container, and the methane fermentation tank may be contained in a second container.

The successive treatment line may include:
a power generator that is contained in a container and generates electric power using methane gas produced in the methane fermentation tank.

The methane fermentation system may include a plurality of the successive treatment lines,
wherein the individual treatment lines treat different types of organic treated materials, and
wherein the methane fermentation system includes a power generator that collects methane gases produced in the individual treatment lines and generates electric power.

### Advantageous Effects of Invention

According to the present disclosure, equipment for producing methane gas from organic waste materials, such as a solubilization tank or a methane fermentation tank, is contained in a container. Therefore, equipment for producing methane gas can be conveyed to different places, achieving ease of move and greater flexibility of installation sites.

### Brief Description of Drawings

FIG. 1 is a schematic diagram illustrating a configuration of a methane fermentation system according to one embodiment of the present disclosure;
FIG. 2 is a schematic diagram illustrating a configuration of the methane fermentation system in which a container holding a power generator is connected; and
FIG. 3 is a schematic diagram illustrating a configuration of the methane fermentation system in which a plurality of different treatment lines is connected.

### Description of Embodiments

One embodiment of the present disclosure will now be described in detail with reference to the drawings.

A methane fermentation system according to the present embodiment produces methane gas through methane fermentation on an organic waste material, and utilizes the produced methane gas as biomass energy. The organic waste material, as used herein, refers to a material that is mainly made up of high-molecular organic compounds such as proteins, carbohydrates, fats, and cellulose, or compounds derived therefrom, and that substantially does not contain solid-state inorganic compounds; provided, however, some solid-state inorganic compounds may be contained to the extent that the inorganic compounds can be easily solubilized and have no adverse effects on the growth of microorganisms.

Examples of the organic waste material include animal sludge, sewage sludge, and kitchen waste like garbage. Such organic waste materials may be concentrated beforehand to have a higher solid concentration to be used as raw materials for solubilization treatment. In addition, an organic material present on a metabolic pathway for methane fermentation treatment, such as a sugar, an organic acid, or a mixture thereof, may be added.

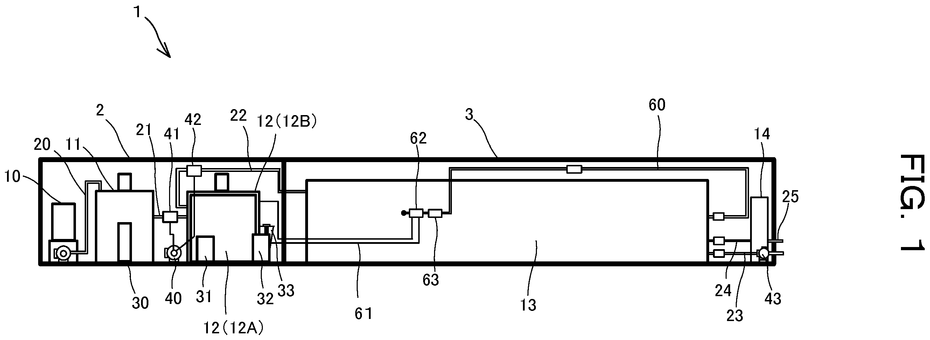

The methane fermentation system 1, in which the above-described organic waste materials are treated, is formed of a body that includes containers 2 and 3 (first and second containers) as illustrated in FIG. 1. The container 2 is a 20-ft container, while the container 3 is a 40-ft container. A successive treatment line is formed of components contained in the containers 2 and 3.

Major contents held in the container 2 include a mohno pump with crusher 10, a mixing tank 11, and a solubilization tank 12. Major contents held in the container 3 include a methane fermentation tank 13 and a desulfurizer 14.

Piping 20 is formed between the mohno pump with crusher 10 and the mixing tank 11. Piping 21 is formed between the mixing tank 11 and the solubilization tank 12. Piping 22 is formed between the solubilization tank 12 and the methane fermentation tank 13. The piping 22 has piping on the container 2 side and piping on the container 3 side that are separated from each other and coupled together with, for example, a joint to form the piping 22. Piping 23 is formed on the methane fermentation tank 13.

An organic waste material is charged into the mohno pump with crusher 10 in the container 2, passes through the piping 20, the mixing tank 11, the piping 21, the solubilization tank 12, the piping 22, the methane fermentation tank 13, and the piping 23 in the order mentioned, and is discharged from the container 3. After discharged, the treated material is utilized as compost.

Piping 24 is formed between the methane fermentation tank 13 and the desulfurizer 14. Piping 25 is formed on the desulfurizer 14.

Into the mohno pump with crusher (crusher) 10, an organic waste material is charged from outside the container 2. The mohno pump with crusher 10 crushes the charged organic waste material into pieces of appropriate size, and sends the crushed material to the mixing tank 11 via the piping 20.

A stirrer disposed inside the mixing tank 11 stirs the charged organic waste material. As a result, the organic waste material contained in the mixing tank 11 is mixed to be in a uniform state. An ultraviolet light irradiator 30 is attached in the mixing tank 11. The ultraviolet light irradiator 30 includes, for example, a light-emitting diode (LED) that radiates ultraviolet light in the mixing tank 11. The ultraviolet light irradiator 30 irradiates the treated material charged into the mixing tank 11 with ultraviolet light to sterilize the material.

A pump 40 and a three-way solenoid valve 41 are attached to the piping 21. Owing to operation of these components, the organic waste material in the mixing tank 11 is sent to the solubilization tank 12 via the piping 21.

In the solubilization tank 12, a solubilization treatment is carried out on the treated material charged therein. For example, a thermal solubilization treatment is carried out with solubilizing bacteria, such as protease-producing bacteria, in the solubilization tank 12. A culture tank (solubilizing bacteria supplier) 31 is attached in the solubilization tank 12. Solubilizing bacteria cultured in the culture tank 31 are supplied from the culture tank 31 to the solubilization tank 12.

An immersion heater 33 is disposed in the solubilization tank 12 in order to heat the inside of the tank. The inside of the solubilization tank 12 is thus kept at a high temperature. A stirrer and a motor are attached in the solubilization tank 12 in order to stir organic waste materials. In addition, an air inlet is disposed in the solubilization tank 12 in order to supply air to organic waste materials. In this way, solubilization is accelerated by stirring or aeration in the solubilization tank 12. High-temperature aerobic refers to the state in which an organic waste material is solubilized at a temperature of 50 to 100°C under normal conditions (in an air atmosphere) preferably without applying pressure.

Solubilization treatment is a treatment by which a solid or water-suspended organic compound generally in a high-molecular state is broken down into a water-soluble organic compound in a low-molecular state. In the present embodiment, protease-producing bacteria are used to carry out ultra-high-temperature solubilization. Protease-producing bacteria are the bacteria capable of producing and secreting a proteolytic enzyme (protease) to the outside of the bacteria.

Examples of the protease-producing bacteria include the genus Bacillus, in particular Bacillus sp. MU3 (Patent Microorganisms Depositary No. NITE AP-156). Being ultra-high-temperature aerobic, the heat-resistant protease-producing bacteria can be fully active at a temperature as high as 80°C. The enzyme produced by the bacteria has a molecular weight of about 57,000, exhibits excellent thermal resistance, and has an outstanding ability to degrade proteins in a wide range of pHs.

The ultra-high-temperature refers to 50 to 100°C, preferably 60 to 90°C, and particularly preferably 70 to 80°C. The ultra-high-temperature solubilization is carried out by bringing an organic waste material in an amount equivalent to a concentration of 50 wt% or less, preferably 5 to 40 wt%, and particularly preferably 10 to 30 wt% into contact with the protease-producing bacteria in an aqueous solution medium, under aerobic or anaerobic conditions, preferably under aerobic conditions. In the present embodiment, under especially preferred conditions, an organic waste material can have a concentration of solids (DS) of the material as high as 10 to 30 wt%, and the solubilization tank is under an optimum aerobic atmosphere desirably at 20% or higher DS, and at a pH of 5 to 8, desirably around 6.

The time period of digestion by protease-producing bacteria is 12 to 72 hours, and preferably 24 to 48 hours. When the digestion takes place under aerobic or anaerobic conditions, the conditions may include stirring and aeration. As described later, under such conditions, ammonia can be removed on site, which means removal of ammonia and solubilization of the material take place simultaneously, achieving accelerated methane fermentation. Bacillus sp. MU3, which is an aerobic and heat-resistant bacterium, is most suitably used as the protease-producing bacteria in terms of both solubilization and ammonia removal because the bacterium enables the material to be solubilized with stirring while being aerated with air.

In the solubilization step according to the present embodiment, bacteria that produce various degradative enzymes other than protease-producing bacteria, such as lipase-producing bacteria, glycositase-producing bacteria, and/or cellulase-producing bacteria, may be added singly or in combination. If these bacteria grow or proliferate under similar conditions, a single reaction tank may be used. If these bacteria grow or proliferate under different conditions, a plurality of solubilization tanks 12 may be disposed, and each solubilization tanks 12 is used under different conditions. In this case, it is preferable to carry out solubilization in the solubilization tanks where lipase-producing bacteria, glycositase-producing bacteria, and/or cellulase-producing bacteria are used, and then carry out solubilization in the high-temperature solubilization tank by using protease-producing bacteria.

An ammonia absorption tank 32 is disposed in the solubilization tank 12. The ammonia absorption tank 32 is disposed in order to absorb and remove ammonia to be generated during a solubilization treatment in the solubilization tank 12.

A pump 40 and a three-way solenoid valve 42 are also disposed on the piping 22. Owing to operation of these components, the treated material solubilized in the solubilization tank 12 is sent to the methane fermentation tank 13 via the piping 22. In the methane fermentation tank 13, the treated material contained therein undergoes a methane fermentation treatment.

Methane fermentation treatment utilizes digestion by methanogens that are usually active in an anaerobic atmosphere. A typical activation temperature for methanogens ranges from 0 to 70°C. Most methanogens will be killed at a temperature higher than the upper limit, although some bacterial strains may survive up to about 90°C. Concerning a lowest temperature range, it is believed that methanogens can survive up to 3 or 4°C. The rate of methane gas production is greatly affected by the activation temperature.

As the fermentation tank is at a higher temperature, a gas is produced faster and in a larger amount. Actually, the following three temperature ranges are found to be livable for methanogens: (1) a low-temperature range of 20°C or lower; (2) a middle-temperature range of 25 to 35°C; and (3) a high-temperature range of 45°C or higher. Although any of the low-temperature, middle-temperature, and high-temperature ranges may be applied to methane fermentation of the present disclosure, methane fermentation at a high temperature of 40 to 70°C is preferable, and methane fermentation at 50 to 55°C is further preferable.

When an organic waste material is subjected to methane fermentation treatment by employing a dry treatment process (where the concentration of charged solids is at least 10%), efficient methane fermentation treatment can be achieved on the organic waste material that has been solubilized at high temperature, and the dry treatment process may be applied to the present embodiment.

In the present embodiment, solubilization treatment can be carried out under high-temperature aerobic conditions, while methane fermentation treatment can be carried out under anaerobic conditions. Therefore, with regard to temperature, the present embodiment is advantageous in that the methane fermentation can be done at a high temperature, which is provided in the solubilization treatment and, with regard to conditions for bacterial growth, the present embodiment is advantageous in that the bacteria subjected to the solubilization treatment (aerobic conditions) are inactivated in the methane fermentation step (anaerobic conditions), and thus do not interfere with the methane fermentation.

In the present embodiment, high-temperature aerobic, as mentioned above, refers to the state in which an organic waste material is solubilized at a temperature of 50 to 100°C under normal conditions (in an air atmosphere) preferably without applying pressure.

The material that has been treated in the methane fermentation tank 13 is discharged from the piping 23 through the use of the pump 43. After discharged, the treated material can be used as compost.

The gas produced in the methane fermentation tank 13 is output to the desulfurizer 14 via the piping 24. The desulfurizer 14 desulfurizes the input gas to extract methane gas, which is then output to the outside of the container 3 via the piping 25.

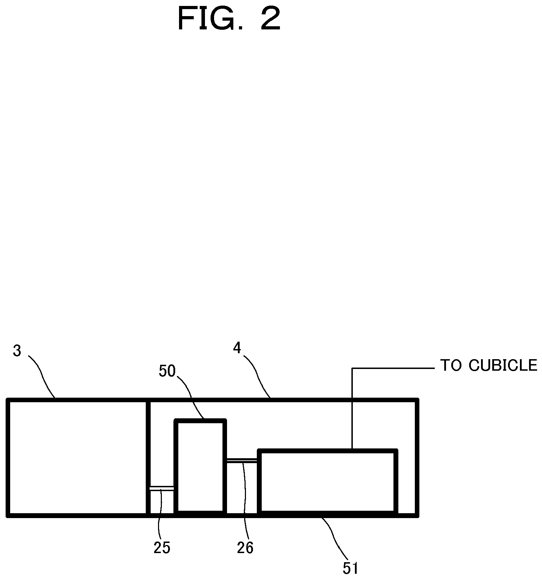

After output to the outside of the container 3, the methane gas is used for electric power generation. For example, as illustrated in FIG. 2, the methane gas may be sent to a power generation unit (a power generator) contained in the container 4 for generating electric power. The container 4, which is a 20-ft container, contains a gas holder 50 and a gas engine 51. The gas holder 50 retains the methane gas sent from the container 3 via the piping 25. The gas engine 51 generates electric power by using the methane gas sent from the gas holder 50 to turn a gas turbine. The electric power generated by the gas engine 51 is sent to a cubicle (a power receiving unit).

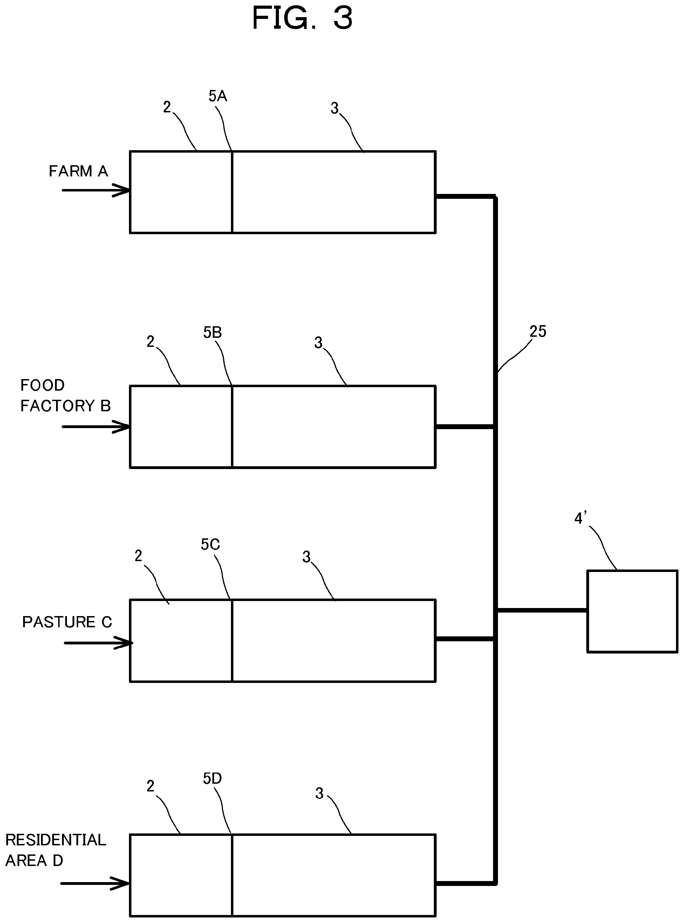

Alternatively, as illustrated in FIG. 3, a plurality of treatment lines each formed of the containers 2 and 3 may be arranged, and methane gases output from the containers 3 in the respective treatment lines may be collected and stored in a single power generation unit 4' for generating electric power. The power generation unit 4' has substantially the same configuration as the container 4 in FIG. 2, but can generate a larger amount of electric power than the container 4 intended for collecting a large amount of methane gas.

For example, in such system, organic waste materials may be separated and charged into the individual treatment lines. For example, as illustrated in FIG. 3, sources of waste may be predetermined as follows: organic waste materials from a farm A are charged into the treatment line 5A, organic waste materials from a food factory B are charged into the treatment line 5B, organic waste materials from a pasture C are charged into the treatment line 5C, and organic waste materials from a residential area D are charged into the treatment line 5D. This allows each individual treatment line to have, to the extent possible, a constant percentage of high-molecular compounds such as proteins, carbohydrates, fats, and cellulose, or compounds derived therefrom, as included in the charged organic waste material.

Assuming that the percentage of compounds included in the charged organic waste materials is variable, the number of fermentative bacteria in the methane fermentation tank 13 may be significantly decreased, leading to a decrease in the amount of methane gas production. Accordingly, by keeping the percentage of compounds in organic waste materials constant, methane-producing fermentative bacteria in the methane fermentation tank 13 are kept ready for producing methane gases through fermentation. As a result, the efficiency of methane gas production is stabilized in each of the treatment lines 5A to 5D, thereby achieving stable supply of methane gases to the power generator in the power generation unit 4'.

Note that the treatment lines 5A to 5D each formed of the containers 2 and 3 may not necessarily be close to one another, and these treatment lines may not necessarily be connected to the power generation unit 4' via piping. The treatment lines 5A to 5D may be placed to be distant from one another, and a gas delivery vehicle may be used to carry methane gases produced in the individual treatment lines 5A to 5D to the power generation unit 4'.

Furthermore, as illustrated in FIG. 1, the solubilization tank 12 according to the present embodiment has an inner container 12A and an outer container 12B disposed therein, where the inner container 12A is to contain the treated material and the outer container 12B is disposed so as to surround the inner container 12A. The treated material to undergo solubilization treatment is contained in the inner container 12A. A heating medium is contained between the outer container 12B and the inner container 12A. In the present embodiment, hot water is used as the heating medium.

In addition, the methane fermentation system 1 includes a treated material circulator 60 disposed therein that circulates the treated material with a pump 63 by taking out the treated material from the methane fermentation tank 13 and returning the material to the inside. Furthermore, the methane fermentation system 1 includes a heating medium circulator 61 disposed therein that circulates the heating medium by taking out the heating medium (water) from the solubilization tank 12 and returning the heating medium to the inside.

The treated material circulator 60 and the heating medium circulator 61 are partially in contact with each other. A heat exchanger device (a heat exchanger) 62 is disposed in the contact region. The heat exchanger device 62 exchanges heat between the heating medium circulating in the heating medium circulator 61 and the treated material circulating in the treated material circulator 60.

The heat exchanger device 62 includes treated material piping, which is part of the treated material circulator 60, and also includes heating medium piping, which is part of the heating medium circulator 61 and surrounds the treated material piping. The individual piping is formed of a highly thermal-conductive material, such as copper. Heat is exchanged between the treated material piping and the heating medium piping. After heated by the heating medium, the treated material returns to the methane fermentation tank 13 to undergo methane fermentation again at a temperature suitable for highly efficient fermentation. By using the heat exchanger device 62 as described above, the treated material to undergo fermentation in the methane fermentation tank 13 is kept at a constant temperature, and thus the efficiency of methane fermentation caused by fermentative bacteria is maintained so as not to be lowered.

As described above in detail, according to the present embodiment, equipment for producing methane gas from organic waste materials, such as a solubilization tank 12 and a methane fermentation tank 13, is contained in containers 2 and 3. Therefore, the equipment for producing methane gas can be conveyed to different places, achieving ease of move and greater flexibility of installation sites.

In the present embodiment, the treated material contained in the methane fermentation tank 13 is kept at a constant temperature with heat generated by a solubilization treatment carried out in the solubilization tank 12. This eliminates the need for providing a hot-water storage tank that stores hot water for keeping the methane fermentation tank 13 at a constant temperature, and thus the whole system can be made smaller. Such ingenuity makes it easier to move the system.

In the present embodiment, the treated material contained in the methane fermentation tank 13 is kept at an appropriate temperature by using the hot water heated in the solubilization tank 12 as the heating medium. In addition to the hot water, the heat produced by the gas engine 51 generating electric power in the container 4 or in the power generation unit 4' may be used to keep the treated material contained in the methane fermentation tank 13 at an appropriate temperature.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2016-60637, filed on March 24, 2016, the entire disclosure of which is incorporated by reference herein.

### Industrial Applicability

The present disclosure can be applied to methane gas production for, for example, biomass power generation.

### Reference Signs List

- 1: Methane fermentation system

- 2, 3, 4: Container
- 4': Power generation unit
- 5A, 5B, 5C, 5D: Treatment line
- 10: Mohno pump with crusher
- 11: Mixing tank
- 12: Solubilization tank
- 13: Methane fermentation tank
- 14: Desulfurizer
- 20, 21, 22, 23, 24, 25, 26: Piping
- 30: Ultraviolet light irradiator
- 31: Culture tank
- 32: Ammonia absorption tank
- 33: Heater
- 40: Pump
- 41, 42: Three-way solenoid valve
- 43: Pump
- 50: Gas holder
- 51: Gas engine
- 60: Treated material circulator
- 61: Heating medium calculator
- 62: Heat exchanger device

## Claims

1. A methane fermentation system comprising a successive treatment line, the treatment line comprising:
a solubilization tank contained in a container, in which tank a solubilization treatment is carried out on an organic treated material; and
a methane fermentation tank contained in a container, in which tank methane is produced by fermenting the treated material that has been solubilized in the solubilization tank,
wherein the solubilization tank comprises:
an inner container that contains a treated material; and
an outer container that is disposed outside the inner container and contains a heating medium heated to a predetermined temperature by heat generated by the solubilization treatment on the treated material contained in the inner container, the heating medium being contained between the outer container and the inner container, and
wherein the methane fermentation system comprises:
a treated material circulator that circulates a treated material by taking out part of the treated material contained in the methane fermentation tank from the methane fermentation tank and returning the part of the treated material to an inside;
a heating medium circulator that circulates the heating medium by taking out part of the heating medium from the solubilization tank and returning the part of the heating medium to an inside; and
a heat exchanger that exchanges heat between the heating medium circulating in the heating medium circulator and the treated material circulating in the treated material circulator.

2. The methane fermentation system according to claim 1, wherein the successive treatment line comprises:
a crusher that is contained in the container and crushes a plurality of different organic waste materials that have been charged; and
a mixing tank that is contained in the container, and contains and mixes the crushed organic waste materials as the treated material to be sent to the solubilization tank.

3. The methane fermentation system according to claim 2, wherein the successive treatment line comprises:
an ultraviolet light irradiator that irradiates the treated material contained in the mixing tank with ultraviolet light.

4. The methane fermentation system according to claim 3, wherein the successive treatment line comprises:
a solubilizing bacteria supplier that supplies solubilizing bacteria to the treated material contained in the solubilization tank.

5. The methane fermentation system according to any one of claims 1 to 4, wherein
the solubilization tank is contained in a first container, and
the methane fermentation tank is contained in a second container.

6. The methane fermentation system according to any one of claims 1 to 5, wherein the successive treatment line comprises:
a power generator that is contained in a container and generates electric power using methane gas produced in the methane fermentation tank.

7. The methane fermentation system according to any one of claims 1 to 5, comprising a plurality of the successive treatment lines,
wherein the individual treatment lines treat different types of organic treated materials, and
wherein the methane fermentation system comprises a power generator that collects methane gases produced in the individual treatment lines and generates electric power.
